# EUROPEAN PATENT SPECIFICATION

(11) **EP 3 873 588 B1**
(45) Date of publication and mention of the grant of the patent: **09.03.2022**
(21) Application number: 19794568.6
(22) Date of filing: 29.10.2019
(51) Int. Cl.: A61N 1/36

(54) **ARTIFICIAL VISION SYSTEM**
KÜNSTLICHES SEHSYSTEM
SYSTÈME DE VISION ARTIFICIELLE

(30) Priority: 30.10.2018 EP 18382763
(43) Date of publication of application: 08.09.2021
(73) Proprietor: Institut de Física d'Altes Energies, 08193 Bellaterra (ES)
(72) Inventor: CHMEISSANI RAAD, Mokhtar, 08193 Bellaterra (ES); MACÍAS MONTERO, Jose Gabriel, 08193 Bellaterra (ES); VERDAGUER MALLORQUÍ, Martí, 08193 Bellaterra (ES)
(74) Representative: de Rooij, Mathieu Julien
(86) International application number: PCT/EP2019/079522
(87) International publication number: WO 2020/089222

(56) References cited:
- US-A1- 2004 181 265
- US-A1- 2010 249 877
- US-A1- 2012 259 410
- US-A1- 2017 368 351

## Description

The present disclosure relates to visual prostheses, and particularly retinal implants.

The present disclosure further relates to artificial vision systems comprising retinal implants. And also relates to methods for implanting the retinal implant and to methods for providing artificial vision.

### BACKGROUND

Retinitis pigmentosa (RP) is a genetic disorder of the eyes that causes loss of vision. Symptoms include trouble seeing at night and decreased peripheral vision (side vision).

The underlying mechanism involves the progressive loss of photoreceptor cells in the back of the eye. In severe cases, a person goes blind because the photoreceptor cells are no longer sensitive to light. However, in most cases the neurons (Retina Ganglion Cell) of the retina are still functional.

Such neurons can be stimulated with an electrical signal, and if such neurons are stimulated, the optical nerve transfers such stimulation to the visual cortex.

US 2012/259410 discloses an apparatus for use with an external non-visible light source is provided. The apparatus comprises an intraocular device configured for implantation in a human eye, and comprising an energy receiver. The energy receiver is configured to receive light emitted from the external non-visible light source, and extract energy from the emitted light for powering the intraocular device. The intraocular device is configured to regulate a parameter of operation of the intraocular device based on a modulation of the light emitted by the external non-visible light source and received by the energy receiver.

US 2017/368351 discloses an intraocular apparatus for implantation in a subject's eye. The intraocular apparatus includes a photosensor array including a plurality of photosensors configured to receive an ambient image, and a power source, for powering the apparatus. The intraocular apparatus additionally including a flexible 0.4-3 mm electrical connector, connecting the photosensor array to the power source. US2004/181265 discloses a self-sufficient retinal prosthesis powered by intra-ocular photovoltaic cells illuminated only by ambient light is provided. Photovoltaic cells can be disposed at the periphery of the retina or in the anterior chamber of the eye. An adaptive retinal prosthesis is also provided, such that the number of pixels energized in the prosthesis is selected according to the variable available power from ambient light.

US 2010/249877 discloses nanoscale photovoltaic devices fabricated from nanoscale waveguides that receive, propagate, and convert incident light into electrical neural signals, and methods of using these photovoltaic devices for visual perception are disclosed herein. A visual neuroprosthetic device includes an array of nanoscale waveguides, each nanoscale waveguide in the array having a photovoltaic material located between an internal conductor and an external conductor, wherein each nanoscale waveguide receives, propagates, and converts incident light into electrical neural signals.

It is known in the art to use a retinal implant intended to provide electrical stimulation of the retina to induce visual perception in blind individuals and it is indicated for use in patients with severe to profound retinitis pigmentosa. A miniature video camera housed in the patient's glasses captures a scene. The video is sent to a small patient-worn video processing unit where it is processed and transformed into instructions that are sent back to the glasses via a cable. These instructions are transmitted wirelessly to an antenna in the retinal implant. The signals are then sent to the electrode array, which emits small pulses of electricity. These pulses stimulate the retina's remaining cells, which transmit the visual information along the optic nerve to the brain, creating the perception of patterns of light. Patients learn to interpret these visual patterns with their retinal implant.

The electrical signals are generated outside the sclera and sent via a cable to inside the eye. Each electrode generates an electric signal independently to excite the neurons underneath the retina's surface.

In one known implant, the electrode array comprises 60 electrodes. Even though patients may recognize very basic shapes thanks to the 60 electrodes, it is clear that a patient may benefit from an increase in electrodes, and from an increased density of electrodes (i.e. electrodes per square area). More neurons or areas might be excited and thus more detailed information on shapes might be transmitted to the optical nerve, potentially leading to improved vision.

However, an increase in the number of electrodes means an increase in power consumption and also in the size of data transfer to configure the matrix of electrodes for each frame image. It is evident that to increase the number of electrodes and still provide them with sufficient power is not a trivial matter.

Moreover, a significant increase of electrodes with good signal quality is also hard to realize due to the increase in electrical wires necessary.

The present disclosure provides examples of systems and methods that at least partially resolve some of the aforementioned disadvantages.

### SUMMARY

In a first aspect, an artificial vision system according to claim 1 is provided. The artificial vision system comprises a visual prosthesis, and particularly a retinal implant, and further comprises a camera, an image processor for analysing a video signal from the camera to generate a data pattern, and a light source for emitting light according to the generated data pattern.

The visual prosthesis comprises an integrated circuit comprising a plurality of pixels, and an array of electrodes attached to the pixels and configured to excite neurons in a retina. The prosthesis furthermore comprises one or more photovoltaic cells, and a photodetector, and the integrated circuit is configured to process signals form the photodetector to selectively activate the pixels.

A functional kit is thus provided for operating the visual prosthesis.

According to this aspect, the retinal implant can be provided with many more individual electrodes or pixels which can increase the quality of vision for patients, as more detailed shapes may be excited. The photovoltaic (PV) cells can be powered by incoming lights. Incoming light flashes or pulses may be registered by the photodetector and the integrated circuit can analyse the received pulses and transform into a selective activation of the pixels. Incoming light can thus both be used to power the implant and to transmit information that might be obtained from a camera.

Moreover, signal quality can be maintained even if the number of pixels increases as the electrodes may be directly attached to the pixels of the integrated circuit. Crosstalk between wires can be effectively avoided.

In the present disclosure, a visual prosthesis may be regarded as any visual device intended to (partly) restore functional vision in those suffering from partial or total blindness.

In particular, the visual prosthesis may be a retinal implant. In the present disclosure, the term retinal implant is meant to cover epiretinal implants (i.e. implants which in use are positioned on the retina), subretinal implants (which are to be implanted behind the retina), and suprachoroidal implants (which are to be implanted between the choroid and the sclera).

In some examples, the visual prosthesis (retinal implant) may comprise a capacitor, and optionally the capacitor is a graphene layer. The capacitor allows the storage of electrical energy derived from the PV cells.

In some examples, the integrated circuit may be an ASIC, i.e. a chip specifically developed for the specific use as herein described.

In some examples, the photodetector may be a PIN diode.

In some examples, the visual prosthesis may be configured to assume an unfolded state and a folded state. The provision of PV cells in the visual prosthesis means that the size of the implant is increased. This could lead to a more problematic process of implanting it. This can be at least partially resolved when the visual prosthesis can be implanted in a folded state, and once in an appropriate position within the eye, the implant can unfold.

One way of implementing the ability to fold or to deploy is by using shape memory material. Shape memory material may be activated in a variety of ways, e.g. if the material is exposed to a change in temperature.

In some examples, the light source may comprise a LED. Specifically, the LED may be configured to emit light with a wavelength in a range of 700 nm or more, particularly 750 nm or more, more particularly 800 nm or more. At these wavelengths, the light can be transmitted through the patient's eye (including lens, cornea and vitreous humor) without significant losses. A retinal implant may thus be used. In particular, the implant may be an epi-retinal implant, i.e. positioned within the retina. Moreover, the PV cells, if selected properly may function at high efficiency at these wavelengths.

In other examples, the implant may be a subretinal implant (i.e. behind the retina), or a suprachordial implant (between the choroid and sclera).

In some examples, the camera, the image processor and the light source are mounted on a support, and in particular on glasses. A patient may wear the glasses and the camera is therewith positioned close to the eyes and the images recorded by the camera are recorded from an angle of vision of the natural eyes.

In yet a further non-claimed aspect, a method for implanting a retinal implant is provided. The method comprises: providing a retinal implant according to any of the examples herein disclosed, and making an incision in a sclera. After making the incision in the sclera, the retinal implant may be inserted through the incision, when the retinal implant is in a folded state. After insertion, the retinal implant may be unfolded.

In some examples, the retinal implant may be provided in a sheath, and unfolding the retinal implant comprises removing the sheath.

In yet a further aspect, a non-claimed method for providing artificial vision for a patient is provided. The method comprises recording an image, processing the recorded image to generate a pixelated image, and producing light pulses transmitting the generated pixelated image to a visual prosthesis. The visual prosthesis may be a retinal implant, and more specifically an epiretinal implant.

In accordance with this aspect data transmission using light is used for communication between a visual prosthesis and an image recording system (e.g. a camera or video camera). The light used for data transmission may be in the visual light spectrum, but preferably will be in the infrared range.

### BRIEF DESCRIPTION OF THE DRAWINGS

Non-limiting examples of the present disclosure will be described in the following, with reference to the appended drawings, in which:
Figure 1 schematically illustrates a prior art retinal implant;
Figures 2A - 2D schematically illustrate a first example of a retina implant and an artificial vision system including such a retinal implant;
Figures 3A and 3B schematically illustrates an example of a retinal implant;
Figures 4A - 4G schematically illustrates a method of implanting according to an example; and
Figure 5 schematically illustrates a further example of a retina implant and an artificial vision system including such a retinal implant.

### DETAILED DESCRIPTION OF EXAMPLES

In these figures the same reference signs have been used to designate the same elements.

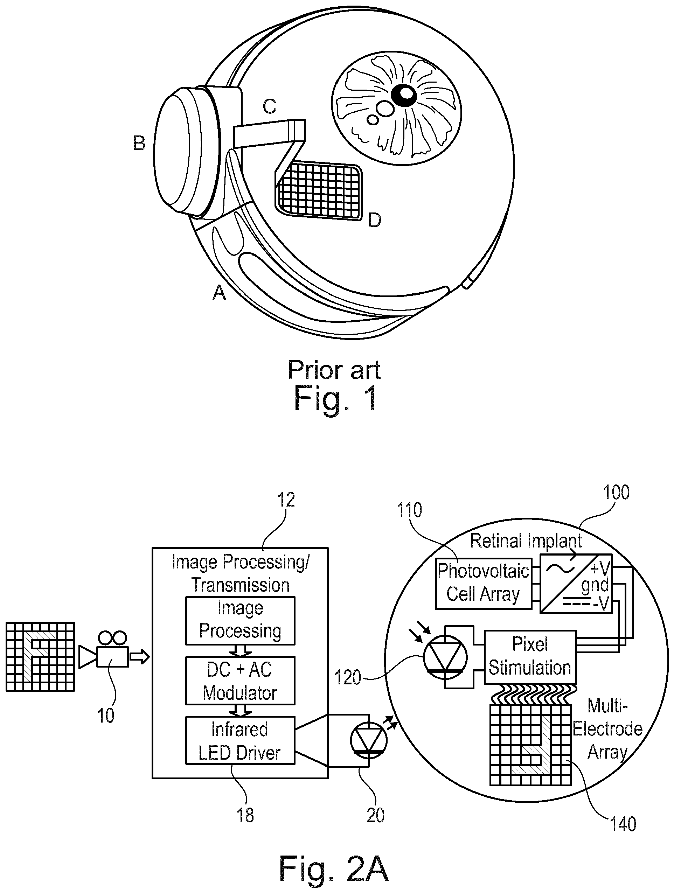

Figure 1 schematically illustrates a prior art retinal implant. The implant comprises an antenna A which receives signals from a further antenna which may be integrated in glasses worn by a user. The signals sent to antenna A may be the result of image processing of a video integrated in the glasses.

Reference sign B refers to an electronic unit outside the eye. Individual electrical cables C lead from the electronics unit to electrode array D.

Figures 2A - 2D schematically illustrate a first example of a retina implant and an artificial vision system including such a retinal implant. The artificial vision system according to this example comprises an implant 100. The implant 100 may be an epiretinal implant.

The system according to this example furthermore comprises a camera 10. The camera 10 may be a digital camera, and specifically a digital camera integrated in glasses that may be worn by a user.

Recordings from camera 10 may be sent in real-time to an image processor 12 for analysing a video signal from the camera to generate a data pattern. The data pattern may be generated by infrared LED driver 18. The LED driver 18 in the example drives the flashing of LED 20. The flashing of LED may have a frequency of e.g. 1 MHz - 1 GHz, specifically 10 - 500 MHz. Both the LED driver 18 and LED 20 may be integrated in the same glasses worn by the user.

The light flashes from LED 20 may be received by a photoreceptor 120 of implant 100. The photoreceptor 120 may be a PIN diode in some examples. Implant 100 includes an integrated circuit, i.e. a chip. Signals received by the photoreceptor 120 may be transformed into excitation signals for the electrodes 140.

In particular, the photoreceptor and circuit may be programmed such that an incoming light flash causes an electric pulse and the electrical pulse is compared to a threshold. If the pulse is above the predetermined thresholds, a "1" is generated. If the pulse is below the threshold (e.g. absent), then a "0" is generated. By sending light pulses at very high frequencies, information is transmitted about the excitation of individual electrodes in electrode array 140. I.e. based on the "1"s and "0"s received, it may be determined which of the electrodes have to be excited.

The implant 100 may be powered by a photovoltaic cell array 110 connected to a suitable converter.

Light is thus used herein both for transmission of information and for transmission of power. There is thus no need for a sizable battery in the implant.

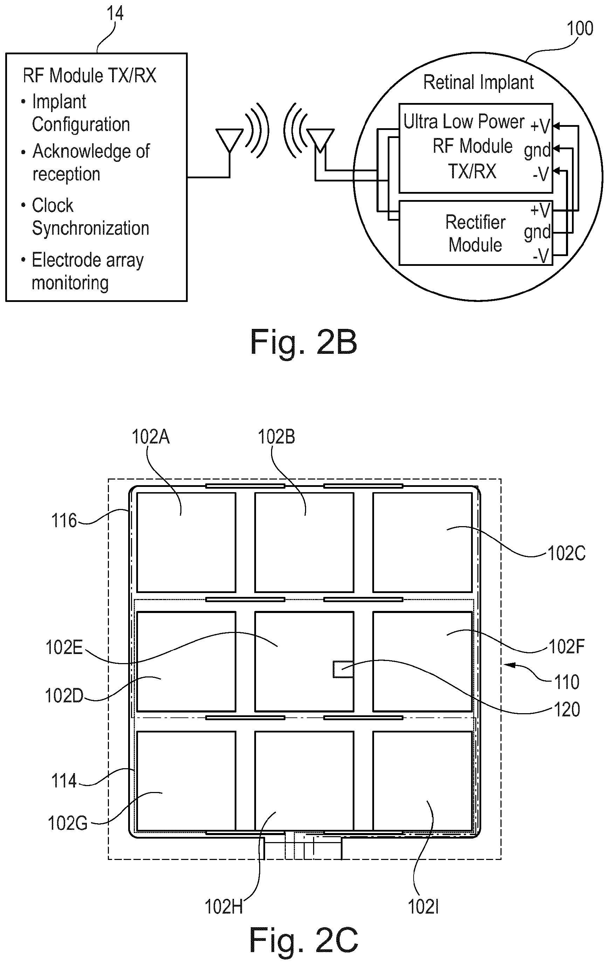

Figure 2B schematically illustrates a high level communication between the glasses 14 (or other support) including the camera, image processor and LED and implant 100. The glasses 14 in this example comprise a first radiofrequency transceiver (rf transmitter/receiver) and the implant 100 comprises a second rf transceiver.

As illustrated in figure 2B, basic functionalities may be communicated through the rf transmitter, including synchronization of the glasses 14 (and thus the video signal and image processing) with the implant (and thus the excitation of individual electrodes), so that the excitation of electrodes is in accordance with the camera recordings. Other basic communications may include e.g. a fault communication, and an acknowledgement of receipt.

Figure 2C schematically illustrates that in this example, the PV array includes 9 PV cells 102A - 1021. In an illustrative example, the PV cells may be PERT cells which have a high efficiency. Each of the PV cells may have an area of 15 - 40 mm², particularly approximately 25 mm². In the depicted example, 9 PV cells of each 5 by 5 mm may be used. The chip with the attached electrodes may also have a size of approximately 5 by 5 mm in this example. The number of pixels and electrodes may be e.g. 15 by 22, 20 by 30, or 25 by 33 or more. As compared to the prior art illustrated in figure 1, the number of electrodes may thus be multiplied by 10 or more. Note that in these examples an approximate ratio of height to width of 2:3 is maintained. Variations with respect to this ratio are possible.

In an example, PERT PV cells may be used with a thickness of approximately 100 microns, e.g. in the range of 50 - 300 microns.

It should be clear that the number of PV cells, the type and the area occupied by the cells may be adjusted in accordance with energy needs. Also another shape may be selected for the PV array.

If the frequency of flashing of the LED is high enough, for example in the aforementioned frequency ranges, the light will be seen as substantially continuous by the PV cells, so that the performance is not negatively affected.

In the central PV cell 102 E, photoreceptor 120 may be integrated.

In an example, the LED 20 may be configured to emit light with a wavelength in a range of 700 nm or more, particularly 750 nm or more, more particularly 800 nm or more. At these wavelengths, the PV cells have a high efficiency, and moreover, the transmission through the human eye (including cornea, lens, vitreous humor) is high as well, for example 85% or more or 90% or more.

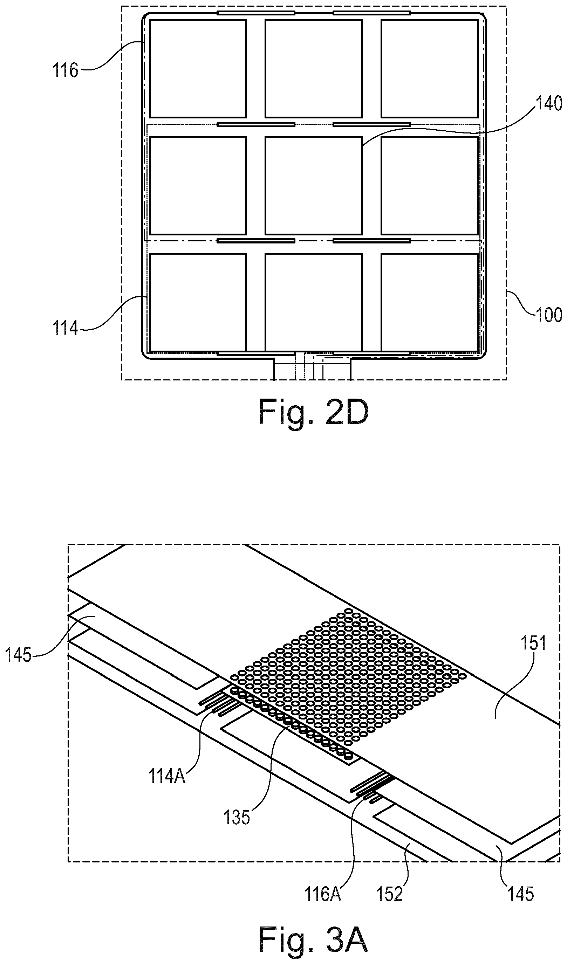

In the example of figure 2D, the retinal implant 100 is configured to assume an unfolded state and a folded state. The implant 100 includes a first frame of shape memory material 114 which when activated forces the implant to the folded shape, and a second frame 116 of shape memory material which when activated forces the implant to the unfolded shape.

In this example, the shape memory material may be thermally activated and particularly may include shape memory metal. The areas around the junctions between the columns of the PV cells may have a memory shape so that the junction is straight or folded. By sending an electrical current through the metal frame 114 or 116, one of the frames may be heated so that the frame has a tendency to assume its memory shape.

If the frame 114 is activated, the junctions between the three columns will fold, such that the implant as a whole assumes the folded shape. If the frame 116 is activated, the junctions between the columns of cells will straighten out, such that the implant will assume an unfolded state.

In examples, the temperature of transition from one shape to another shape may be well above 37ºC, i.e. normal body temperature. If the temperature is chosen close to the average body temperature, a variation of temperature due to e.g. a fever may provoke a change of shape of the implant.

It should be clear that in another example, the tendencies of the shape memory frames may be exchanged. It should also be clear that other shape memory materials and activation methods may be used.

Figure 2D shows the same PV cell array viewed from the opposite side. The bottom sides of the PV cells may be seen. At the bottom of the PV cells, a reflective layer may be provided, e.g. an aluminized mylar^{™} layer, to reflect back any possible incident photon that has passed through the PV cells.

Underneath the central PV cell, the chip with electrode array 140 may be arranged.

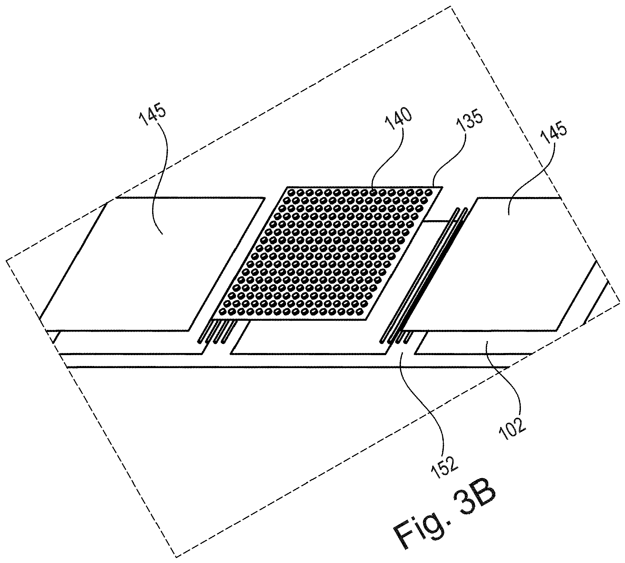

Figures 3A and 3B schematically illustrates an example of a retinal implant. In figure 3, three PV cells are shown, whereas in figure 2 nine PV cells were shown. The illustration in figure 3 may be understood as a separate example of the retinal implant, but it should be clear that the build-up shown in figure 3 may also be regarded as illustrating only a portion of the implant shown in figure 2, i.e. the illustration in figure 3 should not be understood as limiting in size, number of PV cells etc.

The retinal implant comprises a base 152 and the PV cells may be mounted on base 152. The base 152 may be e.g. a polyamide.

The retinal implant further comprises a capacitor 145 to store energy generated by the PV cells. The capacitor 145 may be a graphene layer, and in particular an array of graphene areas 145 connected to each of the individual PV cells. If the same configuration is assumed as in figure 2, there may be nine areas of approximately 5 mm by 5 mm of graphene. The thickness of the graphene layer may be e.g. between 50 and 500 microns. Assuming a thickness of approximately 100 microns, and assuming an estimated capacitance of at least 200 F/g, one may expect to store 18 Joules. If the capacitance of graphene is improved, or the area or thickness of graphene are increased, more energy may evidently be stored.

However, on top of one of the PV cells, an ASIC 135 may be provided. In the configuration of figure 2, the ASIC may be provided on the central PV cell. An array of electrodes 140 may be attached to ASIC 135, in particular the electrodes may be mounted directly, i.e. deposited on the ASIC 135.

In the ASIC, a plurality of pixels may be defined which can be individually excited in accordance with the instructions received from the LED.

In the example of figure 3A, the implant also comprises a top layer 151, and the top layer 151 may be made from polyamide. The top and bottom layers may serve the purpose of reducing the exposure of components of the implant to the surroundings.

In order to allow the electrode to stimulate cells in the eye, the top layer 151 may comprise a plurality of holes corresponding to the electrodes.

Reference signs 114A and 116A illustrate stretches of shape memory material, which may in particular be located between PV cells, and particularly between rows of PV cells, such that the columns may be folded as illustrated in the example of figure 2. The shape memory material may be metallic, and in the illustrated example, the shape memory material may be e.g. Nitinol.

Figure 3B illustrates the same implant (or part of implant) as shown in figure 3A. For the purposes of enhancing the understanding, the top layer 150 has been removed in this figure. The ASIC 135 with electrodes 140 may easily be recognized, as well as graphene areas 145 placed on PV cells 102.

With the build-up of figures 2 and 3 for a foldable implant, the maximum thickness for the implant when folded is preferably below 1 mm. In an example, the thickness of the PV cells is chosen at 100 microns, and the same thickness is chosen for the graphene layer.

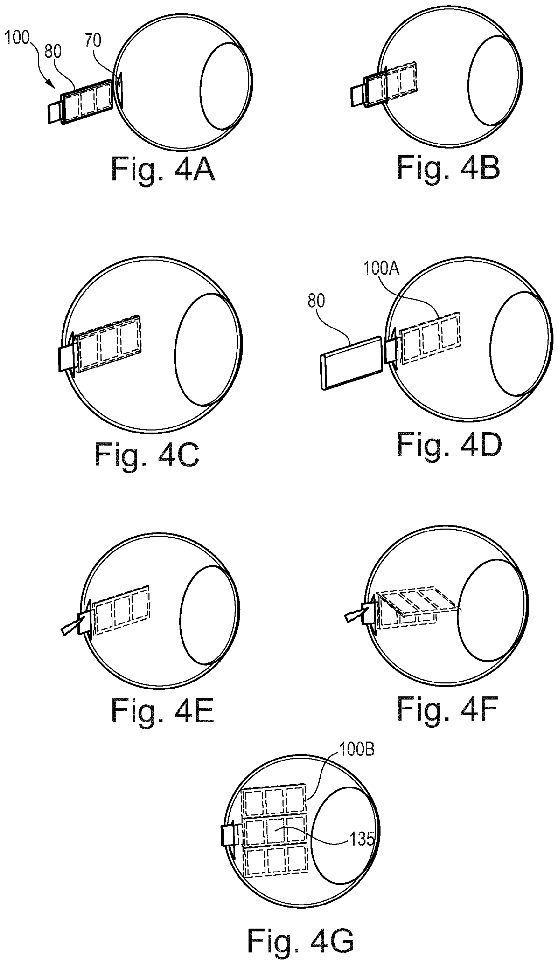

Figures 4A - 4G schematically illustrates a method of implanting according to an example. The implant illustrated in figure 4 substantially corresponds to the implant illustrated particularly in figure 2, but the same method or similar methods may be used in different implants as well.

In figure 4A, an incision 70 may be made in a patient's eye. The implant 100 in this example is provided in a sheath 80. The implant, as will become clear from the following figures, is in a folded state. A small incision is therefore sufficient for the introduction of the implant 100.

Figures 4B and 4C schematically illustrate how implant 100 arranged within sheath 80 is inserted into the eye. In figure 4D, it is shown how sheath 80 may be removed, while the implant is still in the folded state 100A. In figure 4E, an electrical current may be sent through the nitinol wires illustrated in the previous figures. By sending electrical current through the nitinol wires, these wires may heat up and thus assume their remembered shape. As was explained with reference to figure 2, the remembered shape of a frame may be the unfolded or stretched out shape of the wires.

As a result, in figure 4F, implant starts to unfold, to assume in figure 4G the unfolded state 100B. If and when necessary, the implant may be removed by sending electrical current through the other shape memory frame so increase the heat and stimulate that frame to assume its folded shape. Once the implant has assumed its folded state 100A, the implant may be removed. In figure 4G, ASIC 135 (located below the PV cell) is indicated for better reference.

In an alternative implant, and an alternative method for inserting the implant, the implant may be designed to assume its unfolded state without any external stimulation. I.e. merely by removing the sheath 80, the implant will assume the unfolded shape.

It will be clear that the implant, including PV cells, photoreceptor, integrated circuit with pixels attached to electrodes is entirely placed within the eye. The previously illustrated wires for the prior art leading from outside the eye to inside the eye are thus avoided, while energy supply can be ensured.

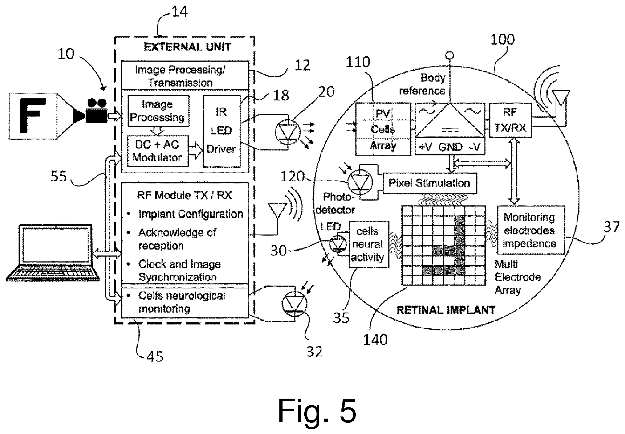

Figure 5 schematically illustrates a further example of a retina implant 100 and an artificial vision system including such a retinal implant. The artificial vision system in this example includes a camera 10, an image processor 12 for analysing a video signal from the camera to generate a data pattern, and a light source 20 for emitting light according to the generated data pattern. The camera 10 and image processor may be arranged on an external unit or support 14, e.g. glasses worn by a patient.

The functioning of the implant 100 and artificial vision system is largely comparable to the functioning of the example in figure 2. The data pattern derived from the video signal after image processing is transmitted to the implant 100. The LED driver 18 in the example drives the flashing of LED 20. Data transmission in this example occurs through light. Photodetector diode 120 receives the light and the data pattern is analysed to stimulate the pixels as in the example of figure 2.

The light flashes from LED 20 may be received by a photoreceptor 120 of implant 100. The photoreceptor 120 may be a PIN diode in some examples. Implant 100 includes an integrated circuit, i.e. a chip. Signals received by the photoreceptor 120 may be transformed into excitation signals for the electrodes 140.

Simultaneously, the light is received by PV cells array 110 to provide power for the functioning of the implant 100.

However, in this specific example, a further functionality is included. If and when a pixel is stimulated, the neurons situated behind and in the immediate vicinity are stimulated. There are however different types of neurons, and upon implanting the retinal implant, the precise disposition of pixels with respect to neurons is not known.

When a pixel is stimulated, not only neurons underlying the specific pixel receive an electric charge, but also neurons underlying neighbouring pixels notice the excitation. By measuring the response at electrodes for pixels neighbouring the pixel that is actually stimulated, neural activity may be derived (block 35) including information on the quantity and types of neurons underlying various pixels.

It may be derived for example, that there is no neural activity under some of the pixels. Exciting these pixels is thus not useful in this case. It may be better to activate a neighbouring pixel instead to approximate the image recorded by the camera. It may also be derived that e.g. neurons underlying some of the pixels are more active than neurons underlying other pixels. If such information can be taken into account in the excitation of the pixels, the visualization of the image recorded by camera 10 may be improved for patients. In some examples, information regarding neural activity may be correlated with specific pixels. In some examples, the information regarding neural activity in relation with pixels may be transmitted to the image processor 12.

In this particular example, the data may be transmitted through light as well. The retinal implant in this example includes a light source 30, e.g. a LED. The light signal emitted from LED 30 may be modulated to transmit data. The light may be received at photodetector 32. The signals received by photodetectors 32 may be analysed (block 45) and the information regarding neural activity in relation with individual pixels/electrodes may be stored and/or fed back (block 55) to the image processor 12. In subsequent image processing to determine appropriate stimulation of pixels/electrodes, the knowledge on neural activity derived from previous stimulations may be used to improve the visual perception of the patient.

The information feedback regarding neural activity (block 35) and transmission through LED 30 may be carried out at a lower frequency than the transmission of light from LED 20. In some examples, information may be gathered during some time before transmitting the information.

In the example of figure 5, (as in the example of figure 2) a high level communication between the glasses (or other support) including the camera, image processor and LED and implant 100. The support 14 in this example comprise a first radiofrequency transceiver (rf transmitter/receiver) and the implant 100 comprises a second rf transceiver.

Basic functionalities may be communicated through the rf transmitter, including synchronization of the glasses (and thus the video signal and image processing) with the implant (and thus the excitation of individual electrodes), so that the excitation of electrodes is in accordance with the camera recordings. Other basic communications may include e.g. a fault communication, and an acknowledgement of receipt. In this particular example, the synchronization also serves to correlate the electrode response that is measured with specific excitations of pixels.

The second rf transmitter may also communicate the electrode impedance of the pixel array and individual pixels. Excitation may be adapted as a function of the measured or determined impedance.

In a further example, the transmission of information regarding neural activity might occur through the second rf transceiver to first rf transceiver on support 60.

Although only a number of examples have been disclosed herein, other alternatives, modifications, uses and/or equivalents thereof are possible. Thus, the scope of the present disclosure should not be limited by particular examples, but should be determined only by a fair reading of the claims that follow.

## Claims

1. An artificial vision system comprising:
a camera (10),
an image processor (12) for analysing a video signal from the camera (10) to generate a data pattern,
a light source (20) for emitting light according to the generated data pattern, and
a visual prosthesis (100), wherein the visual prosthesis (100) comprises
an integrated circuit comprising a plurality of pixels,
an array of electrodes (140) connected to the pixels and configured to excite neurons in a retina,
one or more PV cells (110), and
a photodetector (120) for receiving the light emitted by the light source (20), wherein
the integrated circuit is configured to process signals form the photodetector to selectively activate the pixels.

2. An artificial vision system according to claim 1, wherein each of the electrodes is mounted on one of the pixels.

3. An artificial vision system according to claim 1 or 2, the visual prosthesis further comprising a capacitor, wherein the capacitor is a graphene layer.

4. An artificial vision system according to any of claims 1 - 3, the visual prosthesis further comprising a base layer, the PV cells (110) being mounted on the base layer, and the base layer optionally being made from polyamide.

5. An artificial vision system according to claim 4, the visual prosthesis further comprising a top layer (151), wherein the top layer (151) comprises a plurality of holes (140) corresponding to the electrodes.

6. An artificial vision system according to any of claims 1 - 5, wherein the photodetector (120) is a PIN diode.

7. An artificial vision system according to any of claims 1 - 6, wherein the visual prosthesis (100) is configured to assume an unfolded state and a folded state.

8. An artificial vision system according to claim 7, comprising a first frame (114) of shape memory material which when activated forces the implant to the folded shape, and a second frame (116) of shape memory material which when activated forces the implant to the unfolded shape.

9. An artificial vision system according to any of clauses 1 - 8, wherein the light source (20) comprises a LED.

10. An artificial vision system according to claim 9, wherein the LED is configured to emit light with a wavelength in a range of 700 nm or more, particularly 750 nm or more, more particularly 800 nm or more.

11. An artificial vision system according to any of claims 9 - 10, wherein the LED is configured to emit light pulses at a frequency between 1 MHz and 1 GHz.

12. An artificial vision system according to any of claims 1 - 11, wherein the camera (10), the image processor (12) and the light source (20) are mounted on a support (14), in particular on glasses.

13. An artificial vision system according to claim 12, wherein the glasses comprise a first rf transceiver, and the visual prosthesis comprises a second rf transceiver.

14. An artificial vision system according to claim 13, wherein the first rf transceiver and the second rf transceiver are configured to communicate to synchronize a clock of the glasses with a clock of in the integrated circuit.

15. An artificial vision system according to any of claims 1 - 14, wherein the visual prosthesis further comprises a system for analysing neural activity in response to activation of the pixels.

## Patentansprüche

1. Ein künstliches Sehsystem, das Folgendes umfasst:
eine Kamera (10),
einen Bildprozessor (12) zum Analysieren eines Videosignals von der Kamera (10), um ein Datenmuster zu erzeugen,
eine Lichtquelle (20) zum Emittieren von Licht entsprechend dem erzeugten Datenmuster, und
eine Sehprothese (100), wobei die Sehprothese (100) umfasst
eine integrierte Schaltkreismit einer Vielzahl von Pixeln,
eine Anordnung von Elektroden (140), die mit den Pixeln verbunden und so konfiguriert sind, dass sie Neuronen in einer Netzhaut anregen,
eine oder mehrere PV-Zellen (110), und
einen Photodetektor (120) zum Empfang des von der Lichtquelle (20) emittierten Lichts, wobei
die integrierte Schaltung so konfiguriert ist, dass sie Signale von dem Fotodetektor verarbeitet, um die Pixel selektiv zu aktivieren.

2. Künstliches Sehsystem nach Anspruch 1, wobei jede der Elektroden auf einem der Pixel angebracht ist.

3. Künstliches Sehsystem nach Anspruch 1 oder 2, wobei die Sehprothese ferner einen Kondensator umfasst, der aus einer Graphenschicht besteht.

4. Künstliches Sehsystem nach einem der Ansprüche 1 bis 3, wobei die Sehprothese ferner eine Basisschicht umfasst, wobei die PV-Zellen (110) auf der Basisschicht angebracht sind und die Basisschicht optional aus Polyamid hergestellt ist.

5. Künstliches Sehsystem nach Anspruch 4, wobei die Sehprothese ferner eine Deckschicht (151) umfasst, wobei die Deckschicht (151) eine Vielzahl von Löchern (140) umfasst, die den Elektroden entsprechen.

6. Künstliches Sehsystem nach einem der Ansprüche 1 bis 5, wobei der Photodetektor (120) eine PIN-Diode ist.

7. Künstliches Sehsystem nach einem der Ansprüche 1 bis 6, wobei die Sehprothese (100) so konfiguriert ist, dass sie einen aufgeklappten Zustand und einen zusammengeklappten Zustand annimmt.

8. Künstliches Sehsystem nach Anspruch 7, umfassend einen ersten Rahmen (114) aus Formgedächtnismaterial, der bei Aktivierung das Implantat in die gefaltete Form zwingt, und einen zweiten Rahmen (116) aus Formgedächtnismaterial, der bei Aktivierung das Implantat in die ungefaltete Form zwingt.

9. Ein künstliches Sichtsystem gemäß einer der Klauseln 1 - 8, wobei die Lichtquelle (20) eine LED umfasst.

10. Künstliches Sehsystem nach Anspruch 9, wobei die LED so konfiguriert ist, dass sie Licht mit einer Wellenlänge in einem Bereich von 700 nm oder mehr, insbesondere 750 nm oder mehr, insbesondere 800 nm oder mehr, emittiert.

11. Künstliches Sehsystem nach einem der Ansprüche 9 bis 10, wobei die LED so konfiguriert ist, dass sie Lichtimpulse mit einer Frequenz zwischen 1 MHz und 1 GHz aussendet.

12. Künstliches Sehsystem nach einem der Ansprüche 1 bis 11, wobei die Kamera (10), der Bildprozessor (12) und die Lichtquelle (20) auf einem Träger (14), insbesondere auf einer Brille, montiert sind.

13. Künstliches Sehsystem nach Anspruch 12, wobei die Brille einen ersten HF-Transceiver und die Sehprothese einen zweiten HF-Transceiver umfasst.

14. Künstliches Sehsystem nach Anspruch 13, wobei der erste HF-Transceiver und der zweite HF-Transceiver so konfiguriert sind, dass sie miteinander kommunizieren, um einen Takt der Brille mit einem Takt der integrierten Schaltung zu synchronisieren.

15. Künstliches Sehsystem nach einem der Ansprüche 1 bis 14, wobei die Sehprothese ferner ein System zur Analyse der neuronalen Aktivität in Reaktion auf die Aktivierung der Pixel umfasst.

## Revendications

1. Un système de vision artificielle comprenant :
une caméra (10),
un processeur d'image (12) pour analyser un signal vidéo provenant de la caméra (10) pour générer une schéma de données,
une source de lumière (20) pour émettre de la lumière en fonction de la schéma de données généré, et
une prothèse visuelle (100), dans laquelle la prothèse visuelle (100) comprend
un circuit intégré comprenant une pluralité de pixels,
un réseau d'électrodes (140) connectées aux pixels et configuré pour exciter des neurones dans une rétine,
une ou plusieurs cellules PV (110), et
un photodétecteur (120) pour recevoir la lumière émise par la source de lumière (20), dans lequel
le circuit intégré est configuré pour traiter des signaux provenant du photodétecteur pour activer sélectivement les pixels.

2. Système de vision artificielle selon la revendication 1, dans lequel chacune des électrodes est montée sur l'un des pixels.

3. Système de vision artificielle selon la revendication 1 ou 2, la prothèse visuelle comprenant en outre un condensateur, dans lequel le condensateur est une couche de graphène.

4. Système de vision artificielle selon l'une quelconque des revendications 1 à 3, la prothèse visuelle comprenant en outre une couche de base, les cellules PV (110) étant montées sur la couche de base, et la couche de base étant éventuellement constituée de polyamide.

5. Système de vision artificielle selon la revendication 4, la prothèse visuelle comprenant en outre une couche supérieure (151), dans laquelle la couche supérieure (151) comprend une pluralité de trous (140) correspondant aux électrodes.

6. Système de vision artificielle selon l'une quelconque des revendications 1 - 5, dans lequel le photodétecteur (120) est une diode PIN.

7. Système de vision artificielle selon l'une quelconque des revendications 1 - 6, dans lequel la prothèse visuelle (100) est configurée pour prendre un état déplié et un état plié.

8. Système de vision artificielle selon la revendication 7, comprenant un premier cadre (114) en matériau à mémoire de forme qui, lorsqu'il est activé, force l'implant à prendre la forme pliée, et un second cadre (116) en matériau à mémoire de forme qui, lorsqu'il est activé, force l'implant à prendre la forme dépliée.

9. Système de vision artificielle selon l'une quelconque des clauses 1 à 8, dans lequel la source de lumière (20) comprend une LED.

10. Système de vision artificielle selon la revendication 9, dans lequel la LED est configurée pour émettre de la lumière avec une longueur d'onde dans une gamme de 700 nm ou plus, en particulier 750 nm ou plus, plus particulièrement 800 nm ou plus.

11. Système de vision artificielle selon l'une quelconque des revendications 9 - 10, dans lequel la LED est configurée pour émettre des impulsions lumineuses à une fréquence comprise entre 1 MHz et 1 GHz.

12. Système de vision artificielle selon l'une quelconque des revendications 1 - 11, dans lequel la caméra (10), le processeur d'images (12) et la source de lumière (20) sont montés sur un support (14), notamment sur des lunettes.

13. Système de vision artificielle selon la revendication 12, dans lequel les lunettes comprennent un premier émetteur-récepteur RF, et la prothèse visuelle comprend un second émetteur-récepteur RF.

14. Système de vision artificielle selon la revendication 13, dans lequel le premier émetteur-récepteur RF et le second émetteur-récepteur RF sont configurés pour communiquer afin de synchroniser une horloge des lunettes avec une horloge du circuit intégré.

15. Système de vision artificielle selon l'une quelconque des revendications 1 à 14, dans lequel la prothèse visuelle comprend en outre un système d'analyse de l'activité neuronale en réponse à l'activation des pixels.
